# FASCICULE DE BREVET EUROPEEN

(11) **EP 0 892 626 B1**
(45) Date de publication et mention de la délivrance du brevet: **23.06.2004**
(21) Numéro de dépôt: 97918196.3
(22) Date de dépôt: 10.04.1997
(51) Int. Cl.: A61F 2/06

(54) **DISPOSITIF IMPLANTABLE DESTINE A MAINTENIR OU RETABLIR LA SECTION NORMALE DE PASSAGE D'UN CONDUIT CORPOREL**
IMPLANTIERBARE VORRICHTUNG ZUM FESTERHALTEN ODER WIEDERHERSTELLEN EINES NORMALEN KÖRPERGEFÄSSQUERSCHNITTES
IMPLANTABLE DEVICE FOR MAINTAINING OR RESETTING THE NORMAL SECTION OF A BODY DUCT

(30) Priorité: 10.04.1996 FR 9604443
(43) Date de publication de la demande: 27.01.1999
(73) Titulaire: MINVASYS, 92230 Gennevilliers (FR)
(72) Inventeur: BOUSSIGNAC, Georges, F-92160 Antony (FR); HILAIRE, Pierre, F-75009 Paris (FR)
(74) Mandataire: Hubert, Philippe
(86) Numéro de dépôt international: PCT/FR1997/000635
(87) Numéro de publication internationale: WO 1997/037615

(56) Documents cités:
- EP-A- 0 357 003
- GB-A- 2 092 894
- JP-A- 1 086 983
- US-A- 5 158 548

## Description

La présente invention a pour objet un dispositif implantable destiné à maintenir ou rétablir la section normale de passage d'un conduit corporel.

L'invention trouve principalement application dans le domaine du traitement des vaisseaux sanguins présentant des sténoses.

Elle peut être également avantageusement utilisée dans le traitement des affections de divers conduits du corps humain ou animal, tels que par exemple les conduits urinaires et notamment l'urètre, ou bien encore digestifs et notamment l'oesophage.

Les rétrécissements des conduits sanguins (vaisseaux, artères ou veines) sont à l'origine de troubles graves de la circulation tels qu'en particulier athérosclérose ou phlébites.

Une méthode de traitement de telles affections consiste habituellement à implanter, à l'intérieur du conduit sténosé, un dispositif communément désigné par le terme américain "stent", destiné à rétablir ou à maintenir la section normale de passage dudit conduit au niveau de la sténose.

Ainsi, la fonction d'un tel dispositif est de servir d'étai pour empêcher le conduit de se refermer spontanément, ou bien encore de prévenir son occlusion future par suite de la progression de la maladie athéromateuse.

L'utilisation des "stents" s'est généralisée ces dernières années, et de très nombreux dispositifs ont été proposés dans l'état de la technique.

Ces dispositifs comprennent généralement une armature allongée radialement expansible entre un premier état resserré de diamètre réduit et un second état expansé où celle-ci présente un diamètre sensiblement égal au diamètre naturel dudit conduit corporel à traiter.

On distingue généralement deux grandes catégories de "stents":
les "stents" auto-expansibles, c'est-à-dire propres à passer d'eux-mêmes d'une première position resserrée sous contrainte permettant l'introduction dans le conduit corporel à une seconde position expansée (dispositifs élastiques) ;
les "stents" dont l'expansion est forcée mécaniquement, par exemple au moyen d'un cathéter de dilatation à ballon.

L'invention concerne plus particulièrement un dispositif appartenant à cette dernière catégorie.

Dans leur conformation la plus simple, les dispositifs appartenant à cette catégorie sont constitués d'un ou plusieurs éléments formés d'un fil métallique déformable à faible mémoire élastique enroulé sur lui-même suivant une courbe hélicoïdale.

Un dispositif plus élaboré est par exemple décrit dans le document EP-0.282.175.

Il comprend une armature également réalisée à partir d'un fil métallique déformable ayant une configuration initiale en serpentin, ladite configuration en serpentin étant mise en forme de cylindre ayant un axe horizontal pour former une structure expansible radialement.

Le brevet US 4.886.062 décrit un dispositif analogue à ce dernier réalisé à partir d'un fil ayant une configuration initiale en "zig-zag plat", ladite configuration en zig-zag étant mise en forme de cylindre par enroulement suivant une courbe sensiblement hélicoïdale.

Tous ces dispositifs connus sont donc réalisés à partir d'un fil ayant une configuration initiale plate ultérieurement mise en forme de cylindre.

De telles configurations présentent cependant une capacité d'expansion relativement limitée conduisant à une certaine rigidité axiale qui rend leur mise en oeuvre relativement difficile dans des conduits corporels tortueux tels que par exemple les artères coronaires.

Dans le document JP 64-86 983 sont représentées, à titre d'exemple, diverses configurations de stents sans qu'aucune indication ne soit fournie relativement aux avantages et inconvénients de chacune de ces conformations.

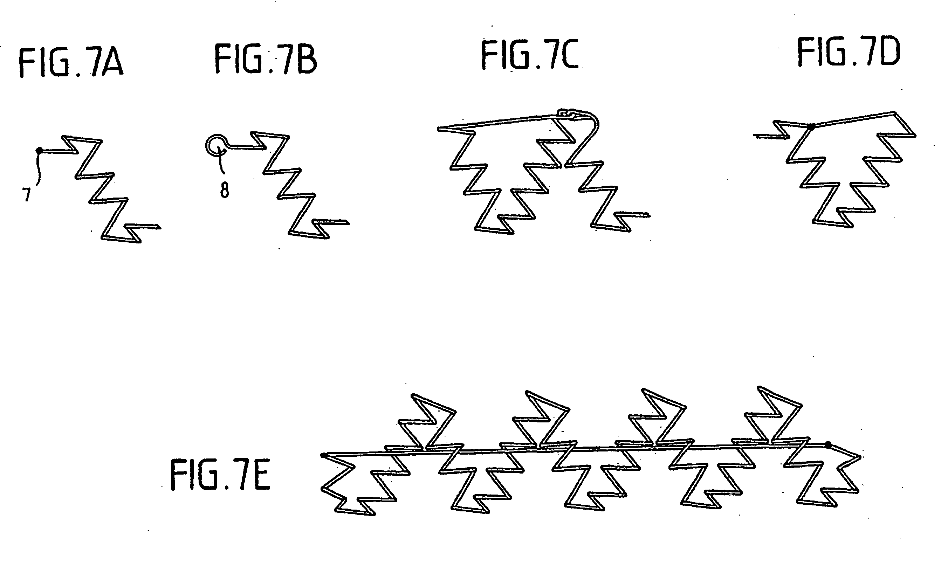

L'exemple illustré par la figure 7E montre un dispositif réalisé à partir d'un fil enroulé une première fois sur lui-même pour présenter une conformation analogue à celle d'un ressort hélicoïdal à spires sensiblement circulaires et une deuxième fois, suivant une ligne directrice sensiblement hélicoïdale.

Un tel dispositif présente une capacité d'expansion plus importante que celle des dispositifs décrits dans les documents EP-0.282.175 et US 4.886.062.

Cependant, ce dispositif présente les inconvénients suivants.

Tout d'abord, en raison de la forme circulaire des spires constituant le premier enroulement (enroulement primaire) ce dispositif présente, à l'état déployé, un encombrement extrêmement important à l'intérieur du vaisseau qui n'est généralement pas souhaitable.

En outre, en raison de la relative souplesse des spires constituant l'enroulement primaire, la mise en place d'un tel dispositif, notamment au moyen d'un cathéter de dilatation à ballon, s'avère difficile. En effet, le gonflement du ballon a tendance à coucher les spires constituant l'enroulement primaire de sorte que le stent, une fois positionné à l'intérieur du conduit corporel, manque de tenue.

Dans ces conditions, la présente invention a pour but de résoudre le problème technique consistant en la fourniture d'un dispositif du type précité, d'une nouvelle conception présentant une capacité d'expansion et une souplesse axiale accrues par comparaison aux dispositifs connus jusqu'à ce jour, et qui puisse être mis en place de façon sûre à l'intérieur d'un conduit corporel sans encombrement de celui-ci.

La présente invention a également pour but de fournir un dispositif qui puisse être réalisé facilement à l'échelle industrielle.

La solution, conforme à la présente invention, pour résoudre ce problème technique consisté en un dispositif implantable destiné à rétablir ou à maintenir la section normale de passage d'un conduit corporel, tel que notamment un vaisseau sanguin, du type comprenant une armature allongée radialement expansible entre un premier état resserré de diamètre réduit et un second état expansé dans lequel elle présente un diamètre sensiblement égal au diamètre naturel dudit conduit corporel, tel que ladite armature comprend à l'état resserré, au moins un élément constitué d'un fil enroulé une première fois sur lui-même, de préférence à pas sensiblement constant, pour présenter une configuration analogue à celle d'un ressort hélicoïdal écrasé, soit ovale, et une deuxième fois suivant une ligne directrice sensiblement circulaire ou hélicoïdale.

Comme on le comprend, l'originalité de la présente invention vis-à-vis des documents EP.0.282.175 et US 4.886.062 réside dans la configuration initiale tridimensionnelle de chaque élément constitutif de ladite armature, par opposition à la configuration initiale bi-dimensionnelle (plate) des dispositifs de l'état de la technique.

Cette configuration initiale tridimensionnelle du fil permet d'obtenir à l'état resserré, un dispositif dont les "spires" sont totalement jointives, ce qui conduit, à encombrement "resserré" égal, à une capacité d'expansion bien supérieure à celle des dispositifs de l'état de la technique.

Cette capacité d'expansion accrue permet de réaliser une armature présentant une excellente souplesse axiale, que celle-ci soit constituée d'un élément unique enroulé sur lui-même suivant une ligne directrice sensiblement hélicoïdale, ou de plusieurs éléments enroulés selon une ligne directrice sensiblement circulaire et reliés entre eux, dans la mesure où il est possible de ménager un espace relativement important entre chaque tour de ladite hélice (cas d'un élément unique) ou entre deux éléments constitutifs (cas d'une pluralité d'éléments).

Un tel dispositif selon l'invention est particulièrement avantageux dans la mesure où il présente à l'état expansé une très grande souplesse tout en conservant un support radial très important.

Vis-à-vis du document JP 64-86983, l'originalité de la présente invention réside dans le fait qu'à l'état déployé, le dispositif induit un encombrement relativement réduit à l'intérieur du conduit corporel. En outre, une fois mis en place, le dispositif conforme à l'invention a une excellente tenue à l'intérieur du conduit corporel.

Enfin, la capacité d'expansion du dispositif conforme à l'invention est encore supérieure à celle du dispositif décrit dans le document japonais précité.

Selon une caractéristique particulière de l'invention, le fil constituant chaque élément de l'armature précitée est réalisé en une matière métallique déformable à faible mémoire de forme.

Avantageusement, une telle matière est choisie dans le groupe comprenant l'acier inoxydable, le tungstène, le platine, le tantale, l'or.

Par ailleurs, le dispositif implantable précité peut être combiné avec un système pour sa mise en place.

Selon une caractéristique particulière, ce système comprend un moyen de dilatation de ladite armature.

Avantageusement, ce moyen de dilatation est constitué d'un cathéter gonflable et l'armature précitée est conformée, à l'état resserré, de telle sorte que ledit cathéter gonflable puisse être reçu à l'intérieur de ladite armature en s'y étendant le long de son axe longitudinal.

Selon une caractéristique particulière, le cathéter gonflable est un cathéter comprenant un ballon plié.

L'invention sera mieux comprise, et d'autres buts, caractéristiques et avantages de celle-ci apparaitront plus clairement à la lecture de la description explicative qui va suivre, faite en référence aux dessins schématiques annexés donnés uniquement à titre d'exemple non limitatif illustrant un mode de réalisation actuellement préféré de l'invention, et dans lesquels:
- La figure 1 est une vue schématique en coupe longitudinale d'un dispositif implantable conforme à un mode de réalisation actuellement préféré de l'invention, et de son système de mise en place à l'état resserré;
- La figure 2 est une vue semblable à la figure 1 du même dispositif et de son système de mise en place au cours de son expansion;
- La figure 3 est une vue semblable aux figures 1 et 2, montrant le dispositif implantable selon l'invention à l'état expansé ;
- La figure 4A représente une vue de côté d'un dispositif implantable selon l'état de la technique représenté par le document EP-0.282.175 ou US 4.886.062, dans sa configuration initiale en zig-zag ;
- La figure 4B est une vue de face de ce même dispositif, à l'état resserré ;
- La figure 4C est une vue de face de ce même dispositif à l'état expansé ;
- La figure 5A est une vue de côté semblable à la figure 4A d'un dispositif implantable selon l'état de la technique représenté par le document JP 64-86983 ;
- La figure 5B est une vue de face semblable à la figure 4B de ce même dispositif, à l'état resserré ;
- La figure 5C est une vue de face, semblable à la figure 4C, de ce même dispositif, à l'état expansé ;
- La figure 6A est une vue de côté semblable à la figure 4A d'un dispositif conforme à la présente invention ;
- La figure 6B est une vue de face semblable à la figure 4B d'un dispositif conforme à la présente invention à l'état resserré ;
- La figure 6C est une vue de face semblable à la figure 4C d'un dispositif conforme à la présente invention à l'état expansé ;
- Les figures 7A, 7B, 7C, 7D et 7E représentent diverses variantes de réalisation de l'extrêmité du fil constituant chaque élément de l'armature d'un dispositif conforme à l'invention.

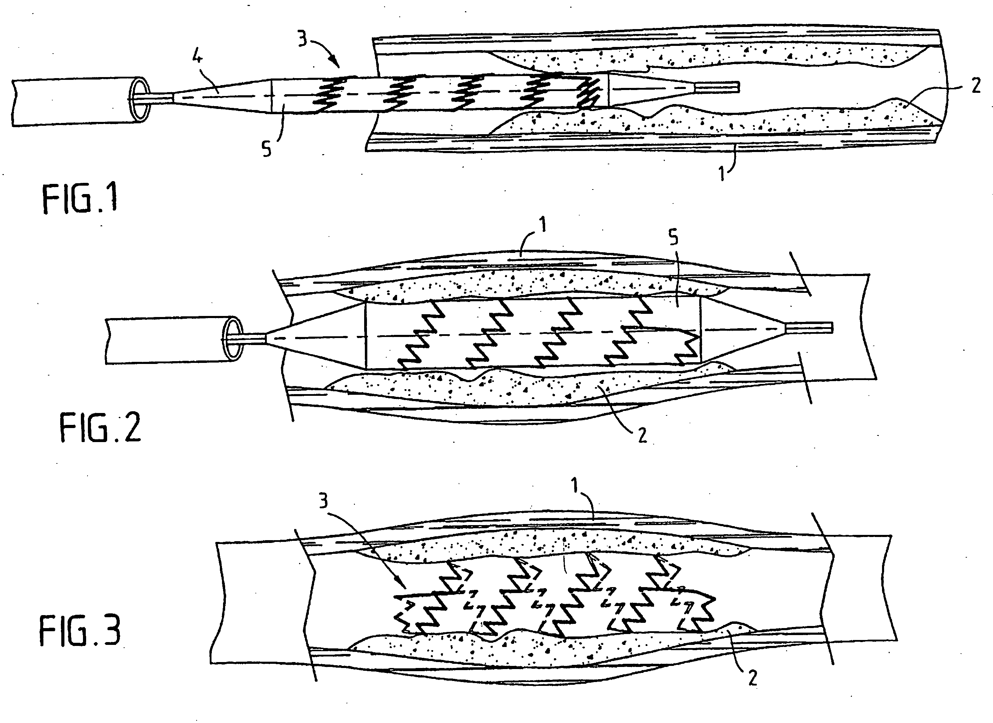

On a donc représenté à la figure 1 un dispositif implantable conforme à un mode de réalisation actuellement préféré de l'invention ainsi que son système de mise en place.

Le conduit corporel choisi à titre d'exemple est un vaisseau sanguin 1 comme en particulier une artère coronaire comportant une sténose 2.

A l'état resserré, représenté à la figure 1, un dispositif selon l'invention comprend généralement une armature allongée 3 radialement expansible comprenant un élément unique constitué d'un fil enroulé une première fois sur lui-même, de préférence à pas sensiblement constant, puis une deuxième fois suivant une ligne directrice sensiblement hélicoïdale.

En d'autres termes, cette structure résulte d'un double enroulement du fil, l'enroulement primaire définissant une configuration initiale tri-dimensionnelle dont les avantages seront explicités ci-après.

Le fil constituant l'armature 3 est avantageusement réalisé en une matière métallique déformable, à faible mémoire de forme, de telle sorte que l'expansion de ladite armature puisse être forcée mécaniquement radialement de l'intérieur vers l'extérieur, par exemple au moyen d'un cathéter de dilatation à ballon.

Avantageusement, une telle matière métallique peut être choisie dans le groupe comprenant l'acier inoxydable, le tungstène, le platine, le tantale, l'or.

L'armature 3 peut être constituée, comme dans l'exemple représenté, par un élément unique, ou par une pluralité d'éléments reliés entre eux, deux à deux successivement.

Dans ce dernier cas, chaque élément peut être configuré sensiblement en "tore" par enroulement suivant une ligne directrice sensiblement circulaire.

La configuration de l'enroulement primaire du fil sur lui-même est analogue à celle d'un ressort hélicoïdal écrasé.

Ainsi, chaque spire de l'enroulement primaire présente une forme sensiblement ovale comme représentée à la figure 6A.

Par ailleurs, la section du fil constituant chaque élément de l'armature 3 peut être rectangulaire, ovale ou de préférence circulaire.

La forme sensiblement ovale de chaque spire de l'enroulement primaire est une caractéristique particulièrement avantageuse du dispositif conforme à l'invention, notamment vis-à-vis de l'état de la technique représenté par le document JP 64-86983, pour des raisons qui seront explicitées plus loin.

Le système de mise en place de l'armature 3 comporte un moyen de dilatation de ladite armature qui est de préférence un cathéter gonflable, généralement désigné par le chiffre de référence 4 et comprenant un ballon gonflable 5.

A l'état resserré, l'armature 3 est conformée de telle sorte que le cathéter gonflable puisse y être reçu, en s'étendant le long de son axe longitudinal.

L'armature 3 peut être expansée, de façon mécaniquement forcée par gonflage du ballon 5, jusqu'à un état où elle présente un diamètre sensiblement égal au diamètre naturel du conduit corporel 1 (voir figure 2).

En raison du fait que la matière constituant le fil est à faible mémoire de forme, l'armature 3 peut alors rester dans sa position expansée.

Dès lors, son système de mise en place peut être retiré comme on le comprend après dégonflage du ballon 5.

On obtient ainsi la conformation représentée à la figure 3.

Afin de mieux faire ressortir l'originalité de la présente invention vis-à-vis des dispositifs de l'état de la technique, on a représenté différentes vues permettant d'établir une comparaison entre un dispositif selon un mode actuellement préféré de l'invention et d'une part un dispositif de l'état de la technique tel que par exemple celui décrit dans le brevet US No. 4.886.062 réalisé à partir d'un fil ayant une configuration initiale en "zig-zag" plat, et d'autre part un dispositif de l'état de la technique tel que celui représenté à la figure 7E du document JP 64-86983.

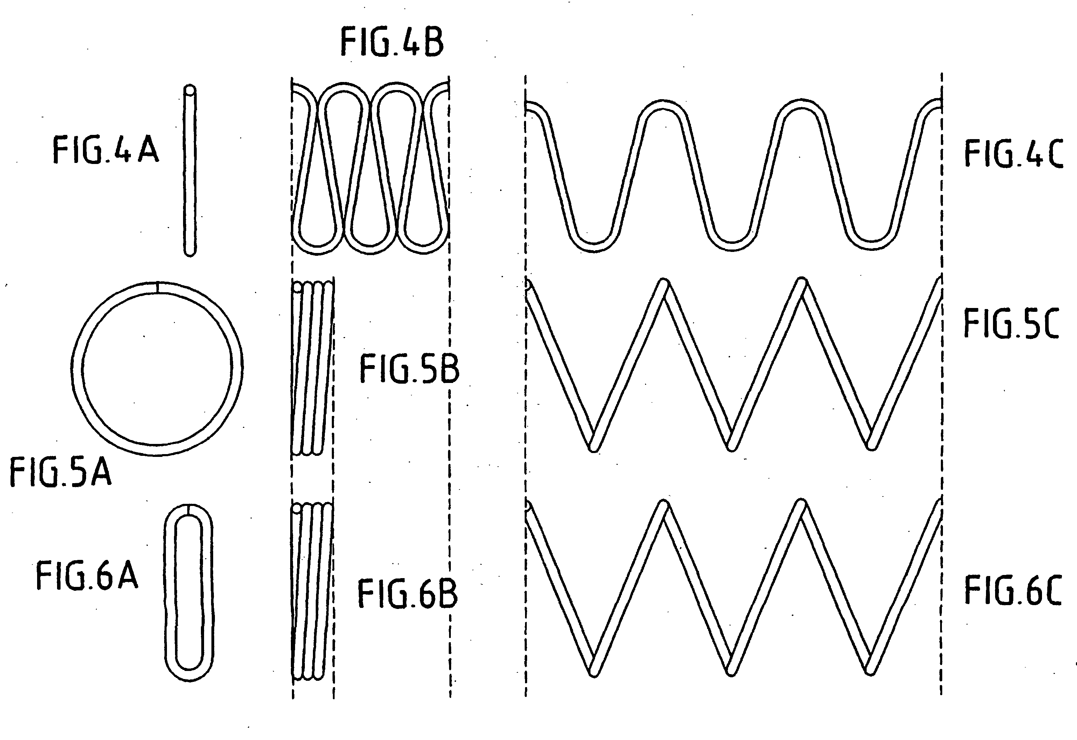

Une comparaison des figures 4A et 6A fait ressortir la configuration initiale tri-dimensionnelle du dispositif conforme à l'invention, par opposition à la configuration initiale plate ou bi-dimensionnelle du dispositif de l'état de la technique.

Une comparaison des figures 4B et 6B fait ressortir immédiatement l'encombrement réduit, à l'état resserré, d'un dispositif conforme à l'invention, par comparaison à un dispositif de l'état de la technique.

Comme on le voit, les spires constituant l'enroulement primaire de l'armature du dispositif conforme à l'invention sont totalement jointives, ce qui diminue de façon importante l'encombrement.

Une comparaison des figures 4C et 6C fait ressortir la capacité d'expansion accrue du dispositif conforme à l'invention.

En fait, l'expansion du fil constituant l'armature d'un dispositif conforme à l'état de la technique se fait uniquement suivant un travail en flexion.

Par opposition, l'expansion du fil constituant le dispositif conforme à l'invention se fait suivant un travail en torsion.

Comme on le comprend, à encombrement resserré égal, le dispositif conforme à la présente invention présente une capacité d'expansion bien supérieure à celle des dispositifs de l'état de la technique.

Cette capacité d'expansion accrue peut être utilisée pour conférer une souplesse axiale au dispositif conforme à l'invention, qui ne peut être obtenue avec les dispositifs de l'état de la technique.

Comme le montre la figure 3, l'espace séparant deux spires consécutives d'un dispositif conforme à la présente invention peut être relativement important tout en permettant de conserver une bonne résistance de l'armature à la compression.

A encombrement resserré égal, les spires consécutives d'un dispositif de l'état de la technique sont pratiquement jointives à l'état expansé, ce qui, comme on le comprend, nuit à la souplesse axiale de la structure.

Par ailleurs, à taux d'expansion égal, le dispositif conforme à la présente invention, nécessite moins de matière métallique pour sa réalisation qu'un dispositif de l'état de la technique.

La capacité d'expansion accrue du dispositif conforme à la présente invention est donc particulièrement avantageuse puisqu'elle permet, pour un taux d'expansion donné, de réduire la quantité de corps étranger dans l'organisme, par comparaison au dispositif de l'état de la technique.

Une comparaison des figures 5A et 6A fait ressortir l'encombrement relativement peu important du dispositif conforme à l'invention relativement à celui décrit dans l'état de la technique constitué par le document JP 64-86983.

En fait, l'encombrement dans le conduit corporel du dispositif conforme à la présente invention est légèrement supérieur à celui d'un dispositif de l'état de la technique tel que représenté par le brevet US 4.886.062 mais bien inférieur à celui d'un dispositif tel que représenté à la figure 7E du document japonais précité.

Une comparaison des figures 5C et 6C fait ressortir la capacité d'expansion accrue du dispositif conforme à l'invention vis-à-vis de celui représenté dans le document japonais précité.

En fait, la forme sensiblement ovale des spires du premier enroulement du dispositif conforme à l'invention permet un travail en torsion relativement important du fil conduisant à une meilleure capacité d'expansion et donc une souplesse axiale supérieure.

En outre, cette forme ovale confère au dispositif conforme à la présente invention une facilité de mise en place et une excellente tenue dans le conduit corporel.

L'extrêmité du fil constituant chaque élément de l'armature 3 d'un dispositif implantable conforme à la présente invention peut être de formes variées.

Cette extrêmité peut par exemple avoir la forme d'une microsphère 7 (figure 7A) ou bien encore être repliée sur elle-même en forme sensiblement circulaire 8 (figure 7B).

Selon une variante de réalisation, l'extrêmité du fil constituant l'armature 3 peut être reliée au tour précédent d'hélice par repliage comme représenté à la figure 7C ou par soudure comme représenté à la figure 7D.

Enfin, selon encore une autre variante de réalisation, les deux extrêmités du fil constituant l'armature 3 peuvent être reliées entre eux suivant un axe sensiblement parallèle à l'axe longitudinal de l'armature (figure 7E).

Le dispositif qui vient d'être décrit peut être mis en place de façon connue en soi, et l'on pourra à cet égard se reporter aux documents de l'état de la technique et notamment au brevet US 4.886.062.

Le dispositif conforme à l'invention peut être en outre appliqué ou utilisé pour la fixation d'implants, en particulier des valves cardiaques ou des membranes élastiques pour l'isolement d'anévrisme:

## Revendications

1. Dispositif implantable destiné à rétablir ou à maintenir la section normale de passage d'un conduit corporel, tel que notamment un vaisseau sanguin (1), comprenant une armature (3) allongée radialement expansible entre un premier état resserré de diamètre réduit et un second état expansé dans lequel elle présente un diamètre sensiblement égal au diamètre naturel dudit conduit corporel, ladite armature (3) comprenant à l'état resserré, au moins un élément constitué d'un fil enroulé une première fois, de préférence à pas sensiblement constant, pour présenter une configuration analogue à celle d'un ressort hélicoïdal, et une deuxième fois suivant une ligne directrice sensiblement circulaire ou hélicoïdale, **caractérisé en ce que** le ressort hélicoïdal formant la structure primaire présente une section ovale.

2. Dispositif selon la revendication 1, **caractérisé en ce que** le fil constituant chaque élément de l'armature (3) précitée est réalisé en une matière métallique déformable, à faible mémoire de forme.

3. Dispositif selon la revendication 2, **caractérisé en ce que** la matière métallique précitée est choisie dans le groupe comprenant l'acier inoxydable, le tungstène, le platine, le tantale, l'or.

4. Système pour rétablir ou maintenir la section normale de passage d'un conduit corporel, tel que notamment un vaisseau sanguin, **caractérisé en ce qu'**il comprend un dispositif implantable selon l'une quelconque des revendications 1 à 3 et un moyen de dilatation de ladite armature (3).

5. Système selon la revendication 4, **caractérisé en ce que** le moyen de dilatation précité est un cathéter gonflable, et **en ce que** ladite armature est conformée, à l'état libre non dilatée, de telle sorte que ledit cathéter gonflable puisse être reçu à l'intérieur de ladite armature en s'étendant le long de son axe longitudinal.

6. Système selon la revendication 4 ou 5, **caractérisé en ce que** le cathéter gonflable est un cathéter comprenant un ballon plié.

## Patentansprüche

1. Implantierbare Vorrichtung zur Wiederherstellung oder Aufrechterhaltung eines normalen Durchlaßquerschnitts eines Körperkanals, insbesondere eines Blutgefäßes (1), mit einer länglichen Bewehrung (3), die radial ausdehnbar ist zwischen einem ersten zusammengezogenen Zustand mit verringertem Durchmesser und einem zweiten ausgedehnten Zustand, in welchem sie einen Durchmesser im wesentlichen gleich dem natürlichen Durchmesser des Körperkanals aufweist, wobei die Bewehrung (3) im zusammengezogenen Zustand mindestens ein Element aufweist, das aus einem Draht besteht, der ein erstes Mal vorzugsweise mit einer im wesentlichen konstanten Ganghöhe gewickelt ist, um eine Struktur analog der einer Spiralfeder aufzuweisen, und ein zweites Mal einer im wesentlichen kreisförmigen oder spiralförmigen Führungslinie folgend gewickelt ist, **dadurch gekennzeichnet, daß** die Spiralfeder, die die erste Struktur bildet, einen ovalen Querschnitt aufweist.

2. Vorrichtung nach Anspruch 1, **dadurch gekennzeichnet, daß** der Draht, der jedes Element der oben erwähnten Bewehrung (3) bildet, aus einem verformbaren metallischen Material mit geringem Formerinnerungsvermögen hergestellt ist.

3. Vorrichtung nach Anspruch 2, **dadurch gekennzeichnet, daß** das oben erwähnte metallische Material aus der Gruppe bestehend aus rostfreiem Stahl, Wolfram, Platin, Tantal, Gold gewählt ist.

4. System zur Wiederherstellung oder Aufrechterhaltung des normalen Durchlaßquerschnitts eines Körperkanals, insbesondere eines Blutgefäßes, **dadurch gekennzeichnet, daß** es eine implantierbare Vorrichtung nach einem der Ansprüche 1 bis 3 und eine Einrichtung zum Erweitern der Bewehrung (3) aufweist.

5. System nach Anspruch 4, **dadurch gekennzeichnet, daß** die oben erwähnte Erweiterungseinrichtung ein aufblasbarer Katheter ist und dadurch, daß die Bewehrung im freien, nicht erweiterten Zustand so passend ist, daß der aufblasbare Katheter im Innern der Bewehrung aufgenommen werden kann, wobei er sich entlang deren Achse erstreckt.

6. System nach Anspruch 4 oder 5, **dadurch gekennzeichnet, daß** der aufblasbare Katheter ein Katheter ist, der einen zusammengefalteten Ballon aufweist.

## Claims

1. An implantable device intended to reestablish or maintain the normal passage cross section of a bodily canal, such as a blood vessel, comprising an elongate framework (3) which can expand radially between a first, contracted state of reduced diameter and a second, expanded state in which it has a diameter substantially equal to the natural diameter of said bodily canal, said framework (3) having, when contracted, at least one element consisting of a wire wound a first time, preferably with substantially constant pitch, to have a configuration similar to that of an helical spring, and a second time, along a substantially circular or helical directrix line, wherein said helicoïdal spring forming the primary winding has an oval section.

2. The device as claimed in claim 1, wherein the wire constituting each element of the aforementioned framework (3) is made of a deformable metallic material, with weak shape memory.

3. The device as claimed in claim 2, wherein said deformable metallic material is selected from the group consisting of stainless steel, tungsten, platinum, tantalum and gold.

4. A system for reestablishing or maintening the normal passage cross section of a bodily canal, such as a blood vessel, which comprises a device as claimed in any of claims 1 to 3, and a means for dilating said framework.

5. The system as claimed in claim 4, wherein the aforementioned dilatation means is an inflatable catheter, and wherein said framework (3) is configured, in the undilated free state, in such a way that said inflatable catheter can be received inside said framework while extending along its longitudinal axis.

6. The system as claimed in claim 4 or 5, wherein the inflatable catheter is a catheter comprising a folded balloon.
